# EUROPEAN PATENT APPLICATION

(11) **EP 1 813 349 A1**
(43) Date of publication of application: **01.08.2007**
(21) Application number: 07001177.0
(22) Date of filing: 19.01.2007
(51) Int. Cl.: B01L 3/00, B01L 3/02, A61B 5/15, G01N 1/34, G01N 33/483, G01N 33/68

(54) **Extraction of substances of interest from blood for mass spectrometric analysis**

(30) Priority: 30.01.2006 DE 102006004165
(71) Applicant: Bruker Daltonik GmbH, 28359 Bremen (DE)
(72) Inventor: Franzen, Jochen, 28359 Bremen (DE); Baum, Hans-Jakob, 28832 Achim (DE); Michelmann, Karsten, 27243 Harpstedt (DE)

(57) **Abstract**

The invention relates to sample preparation for the mass spectrometric determination of substance profiles from blood, the substances to be measured being obtained by extraction with concentration ratios which have the best possible reproducibility. The method is particularly focused on protein profiles.

The invention provides a method of sample preparation in which, immediately after the blood sample has been taken from a subject, the extraction is carried out by means of reversible immobilization on surface layers, and only the extracted substances of interest are sent in the immobilized state to the mass spectrometry laboratory for the substance profile to be measured. The extraction is performed preferably from the fresh whole blood, but blood plasma or blood serum can be used if the production from the fresh blood is done without delay. This largely prevents irreproducible changes of the substance concentrations by chemical or enzymatic processes in the lastingly reactive blood during the time of transport; and it also obviates the need to add enzyme inhibitors or to freeze the blood sample to minus 80°C for transportation to the mass spectrometric laboratory. Profiles of proteins or other substances can be used for the search for disease markers ("biomarkers"), for medical diagnoses, or to check the dosing or effectiveness of medication.

## Description

### Field of invention

The invention relates to sample preparation for the mass spectrometric determination of profiles of substances of interest, preferably of protein profiles, from fresh blood.

### Prior Art

Mass spectrometric diagnostics carried out by measuring and evaluating substance profiles obtained from body fluids is very promising, but still in its infancy. The first mass spectrometers which are licensed for medical diagnostics are now coming onto the market. The licensing is effected by means of a manufacturer's IVD declaration of conformity (CE), which is strictly monitored by official bodies. The abbreviation IVD stands for "in vitro diagnostics". In the United States, for instance, the IVD diagnostics is regulated by the FDA. In Germany, it is regulated by the Medical Devices Act (MPG, Medizin-Produkte-Gesetz), which is based on the European Directive 98/79/EC.

A particularly promising diagnostic method is one which measures protein profiles from blood. Blood is routinely sampled from a vein in every doctor's practice; the risk from side effects is very low. The risk is even smaller when only a drop of blood is taken from the fingertip or earlobe.

Significant over- and underexpressions of specific proteins can be measured in the protein profiles from the blood, said expressions being reflected in concentrations which are too high or too low. Furthermore, chemical changes to proteins can be measured, said changes then appearing with another molecular weight at other points in the mass spectrometric protein profile. Statistically significant changes are always an indication of a specific stress situation of the body, and in some cases are even characteristic of a specific disease or bodily anomaly. Such proteins, which undergo characteristic changes in their concentration or molecular weight as a result of stress, are now termed "biomarkers". The term "biomarker" generally refers not just to an individual protein, but rather to a pattern of several proteins in their concentration ratios relative to each other. Measurement of such biomarkers or biomarker patterns can be used to medically diagnose diseases or anomalies, to dose or monitor medication, and also for many other purposes through to pharmacokinetic analyses.

Analysis procedures of protein profiles, however, are not only used for diagnostic purposes, they are widely used by scientific investigations of the influence of different types of stress on the expression of proteins; worldwide many search programs for biomarkers are under way. Many of these investigations are not performed on human blood, basic information is often gained from experiments with animals.

Blood consists mainly of water (around 90%), various types of blood particles, small amounts of salts, several non-protein organic substances, and around seven per cent is made up of proteins, of which albumins, globulins und fibrinogens form the largest part. The general term "blood samples" below can mean "whole blood", "blood serum" or "blood plasma"; the differences are explained in more detail below. The proteins which are of interest here as possible biomarkers are present in the blood samples at lower concentrations of less than one per cent and down to 10⁻⁸ per cent. The proteins present in very low concentrations elude direct measurement if they cannot be especially "fished out" in a substance-specific way or indirectly measured by the effects they cause.

In a good mass spectrometer, a hundred attomols of a protein (60 million molecules) can still provide a measurable signal; however, this detection limit is considerably higher in a protein profile with its unavoidable background noise. The detection limit then amounts to around ten femtomoles. For a peptide with a molecular weight of 1,000 Daltons, this corresponds to ten picograms, and to a hundred picograms for a protein with a molecular weight of 10,000 Daltons. In a small drop of blood containing only ten microliters of blood (ten milligrams) it is thus possible to measure proteins down to a concentration of 10⁻⁶ per cent if it proves possible to feed all the proteins of interest to the measurement and to measure a protein profile which is not completely overloaded with proteins. Immediately above the detection limit, however, the accuracy of the measurement is not very good, and this generally limits the measurement and evaluation of the protein profiles to a concentration range between 10⁻¹ and 10⁻⁴ per cent.

The smaller proteins with molecular weights of up to several thousand Daltons, which consist of only a few tens of amino acids, are called peptides; they are included here in the term "proteins" however. The dividing line between peptides and proteins is not well defined. The vast majority of peptides in the blood are so-called "digest peptides", which are created as a result of the enzymatic breakdown of large proteins such as fibrinogens, but also of body-foreign proteins, which takes place continuously to a greater or lesser degree. Until very recently, these digest peptides were considered to be "garbage" which contained no information about the state of the body. In a recently published paper it was proven, however, that in blood characteristic patterns of peptides could be measured indicating specific enzymes in each case. These enzymes could, in turn, be unambiguously assigned to specific types of cancers. It was also possible to detect the patterns of these digest peptides in blood serum or blood plasma. The enzymes themselves evaded mass spectrometric measurement because their concentration was much too low; they only revealed themselves through the products of their activity. These peptide patterns are thus indeed suitable as biomarkers, but only if the fresh blood is subjected to a specific treatment for transport and storage to prevent further uncontrolled activity of the enzymes.

The number of different types of proteins in the blood is extremely high, far above 100,000. Even in the measuring range of the protein profiles there are many thousands of proteins. A profile with such a large number of proteins would not allow to identify the individual proteins because the mass spectrometric signals would produce unresolved superimposition. It is therefore necessary to drastically reduce the number of proteins before measuring a protein profile, yet still provide a large number of proteins for the measurement. This is done by means of broadband extractions, which are able to extract proteins which share specific properties. As a rule, such broadband extractions can simultaneously extract several tens to several hundreds of proteins whose concentrations are in the measurable range. Because in many cases the extraction of a small number of substances is likewise of interest, for example, the extraction of only two proteins for a determination of their concentration ratio in a reproducible way, we will use the expression "extraction" without the addition "broadband" to underline this situation. The term "extraction" should be interpreted here as the extraction of at least two substances for a comparative analysis of their concentration.

There are different types of broadband extraction, different with respect to "what" (types of protein extracted) as well as to "how" (extraction mechanism). Reversible immobilization of proteins on suitable actively binding surfaces of solids is the easiest extraction mechanism to work with. It is the only one considered here. The actively binding surfaces of solids are usually produced by permanently coating the surfaces of the solids with suitable substance layers.

The different extraction methods are distinguished by actively binding surface layers which have different binding properties; they usually extract completely different types of protein out of the blood sample. The proteins may be bound, for example, to the surface layers by means of electric interactions, particularly by permanently coating the surfaces with anion or cation exchangers. Such layers extract proteins with different ionic charges from the blood. Other proteins may be affinitively bound via hydrophobic bonds, as occurs in reverse phase chromatography. Other types of proteins again can be held on the surface by means of different types of chelate-type bonds, by ligand bonds, and also by customized, protein-specific bonds along the lines of the antigen-antibody bond. This means that a mixture of different antibodies permanently bound to the surfaces of solids can also extract protein profiles, not only individual proteins.

Different types of extraction usually produce totally different protein profiles because quite different types of proteins are extracted by reversible immobilization on the surface of the solids in each case. Patterns of characteristic biomarkers can also be composed of signals from different types of protein profiles, they therefore do not need to originate from a single protein profile. Furthermore, not only the proteins are of analytical interest for diagnostic or scientific purposes. The procedures for extraction and mixture analysis should therefore not exclude other types of substances from being observed.

The actively binding surface layers for the extractions can be coated on different types of surfaces. On one hand, the vessel walls of the sample containers can themselves be actively coated. On the other hand, actively binding layers can be located on special materials filled into the vessel. All coatings should be permanently, i.e. irreversibly, bound. The blood samples can be forced through actively binding filter material in the form of felt, open-pored foams or particle-filled cavities. Macroscopic beads or pellets, or suspensions of microparticles or nanoparticles with actively binding layers can be added to the liquid sample and later recovered from the sample liquid by filtration, centrifuging or magnetic forces.

One problem of diagnostics using proteins from blood samples is the undesired, continuing chemical conversion of at least some proteins during storage and transportation. Blood is a lastingly reactive liquid; the enzymes it contains do not lose their effectiveness when a blood sample is taken. The enzymes digest other proteins or other substances contained in the blood, or they change them in a characteristic way. In addition, there are chemical processes, for example oxidation, which are often also controlled by enzymes. Another factor is clotting, which is caused by the fibrinogens changing into fibrous fibrin, and which is also controlled by enzymes such as thrombin. Thrombin is formed by the decomposition of the platelets (thrombocytes), which are one type of small blood particles. The speed of alteration of the proteins in the blood depends on many factors: for example, the temperature of the sample is important, as is its state of motion and the individual composition of the blood itself. The blood must therefore always be stabilized for transportation or storage.

A first step in the stabilization is the addition of anticoagulants such as heparin, hirudin, EDTA, or citric acid. This type of coagulant stabilization is only effective for a limited period, however, and presents a considerable interference with the sample. A measure which is therefore usually chosen, and which is effective over a longer period, is the removal of the blood particles, generally by gently centrifuging in centrifuges of the type available in practically all doctor's practices. This leaves the colorless (slightly yellow) "blood serum", which also has to be prevented from clotting, however. If artificially induced clotting is used to remove the fibrinogens, then "blood plasma" is obtained which, in itself, is considered to be very stable, but whose proteins are still continuously altered by enzyme reactions. For longer periods of storage or transportation, it is hence still necessary to add enzyme inhibitors, other chemical stabilizers or antibiotics to the blood serum or blood plasma. The addition of substances is always an adulteration of the original sample, however.

Freezing the blood sample, i.e. the whole blood, the blood serum or the blood plasma, at minus 80°C has therefore established itself as a significantly better type of stabilization method, but it can only be carried out in a small number of doctor's practices; usually only in hospitals. To transport the blood over long distances, carriers who specialize in refrigerated transportation have to be used, making the transportation expensive.

Mass spectrometric diagnostics regularly requires the sample to be transported over a long distance since mass spectrometers are expensive and their operation is complex, and they can only be found in central facilities. Until now, mass spectrometric diagnostics have therefore been geared to freezing at minus 80°C and special transports, a situation which prevents this diagnostic method from becoming more widespread.

The objective of the invention is to provide an economical method of sample preparation for the reproducible measurement of substance profiles from blood samples which include easy sample transportation from the location of blood taken to the mass spectrometry laboratory. This involves taking the blood in scientific laboratories from animals, in doctor's practices from patients, or even at home and measuring the protein profiles in distant mass spectrometric laboratories. The samples containing the sets of substances of interest have to be prepared in such a way that economical transportation of the samples at normal temperature and standard transportation conditions is possible without quantitative and qualitative changes of their composition.

### Summary of the Invention

The basic idea of the invention is to perform the extraction of substances of interest immediately after taking the fresh blood from a subject at the (first) location, by reversible immobilization on actively binding surface layers in preferably ready-made vessels before any severe changes of the composition of the blood take place, and to send the vessel with the extracted and washed substances of interest in a still immobilized state at normal temperature and under normal transport conditions to the (second) location of the mass spectrometry laboratory for analysis. The first location of blood sampling may be a scientific laboratory, a hospital, a doctor's practice, or even a patient's home. The second location of the mass spectrometry lab may be located in the neighboring house, in another quarter of the city, in another town, or even in another state; the immobilized state prevents the substances reacting with each other during any reasonable length and duration of transport. Particularly any enzymatic activity is suppressed. The extraction of the substances of interest from the fresh blood by reversible immobilization on surface layers and the subsequent washing can preferably be performed in the blood sampling vessels using the whole blood, only if the special extraction procedure cannot be carried out with sufficient reproducibility from fresh whole blood, the extraction may take place in special vessels from blood serum or blood plasma produced immediately from the fresh blood after sampling in the first location, e.g. in the doctor's practice.

It is possible to use ready-made vessels with built-in extraction capabilities, either for taking whole blood samples, or for the blood serum or the blood plasma samples, prepared with permanently bound extraction surface layers suitable for extraction of the substances of interest. If different types of surface layers are used, different sets of substances of interest may be extracted. The extraction layers can be located at the interior wall surface of the vessels, for example, or on macroscopic or microscopic packing pellets or other types of packing material such as felt, open-pore plastic foams, or frits. Various types of microscopic or macroscopic packing pellets, distinguishable and separable by their form, magnetic properties, size or color, or different wall or packing zones within the vessels can extract different sets of substances for different types of substance profiles of interest and allow, after removing the sets of immobilized substances separately in the mass spectrometry lab, for separated mass spectrometric measurements of the different substance profiles.

Standard tubular vessels for taking blood samples in doctor' practices are usually delivered in evacuated state and equipped with a membrane, which can be punctured by the backside of a standard blood sampling needle. The vacuum suction fills the vessel with blood. For the extraction of substances of interest from the whole blood, surface layers can then bind the substances of interest. Care must be taken, however, that the blood has as much contact with the surface layers as possible; simple diffusion generally takes too long in the very viscous blood. The intimate contact can be brought about by continuous turning (as happens with an hourglass), for example, or by stirring, for example with magnetic stirring bodies, whose surfaces can also carry actively binding layers.

Specially prepared extraction pipettes, preferably made of plastic, can be used for the broadband extraction of substances of interest from only one drop of blood, for example a drop of blood obtained by pricking the fingertip or earlobe. Said pipettes having a small rubber bulb for drawing up liquids, on the one hand, and internal actively binding layers on the interior wall surface or on packing material, on the other. Repeatedly drawing up the blood produces good surface contact.

In all vessels prepared for extraction in this way, the immobilized substances are subsequently washed repeatedly with a suitable washing liquid. The washing liquid may also be ready-made. The vessels are then sealed with a cap and sent to the mass spectrometric laboratory for analysis. Depending on the method, the washing liquid can remain in the vessel, or the vessel can be filled with a stabilizing liquid which may also be ready-made, or it can be sealed empty in a moist or dried state.

The extraction process and the analysis procedure can be improved by adding special substances to the blood which may be already contained in the ready-made vessel. The substances may avoid clotting of the blood like e.g. heparin, or they may serve as quantitative measurement references for the extraction, immobilization and final removal process for the substances of interest. For different sets of substances, different reference substances may be added.

### Brief Description of the Illustration

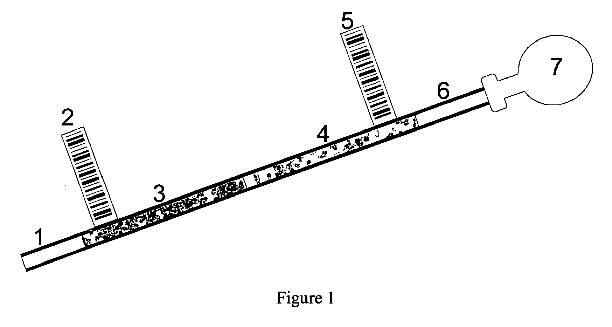

Figure 1 shows an extraction pipette for the extraction of two sets of substances of interest from a drop of blood with rubber bulb (7) and two different packing zones (2) and (5) with corresponding surface layers respectively.

### Particularly Favorable Embodiments

A first favorable, very simple embodiment for the immediate broadband extraction procedure of proteins from a droplet of blood obtained from the fingertip or earlobe when it is pricked consists in the use of an one-way extraction pipette made of unbreakable plastic, as schematically shown in Figure 1. The procedure may be performed in a hospital, in a medical office (doctor's practice or doctor's surgery), or even at home.

The broadband extraction of the substances from whole blood is carried out immediately when the blood is drawn up into the extraction pipette. The interior surface of the extraction pipette comes pre-equipped with the actively binding extraction layers either on the internal pipette body wall, or on packing material. For ease of operation, one end of the extraction pipette may have a small rubber bulb; the other end may be equipped with a tightening cap so that it can be closed for transportation. The extraction pipette may be supplied prefilled with an inert gas to avoid formation of an oxidizing surface.

An extraction pipette not packed with other material, having a length of some eight centimeters and an internal diameter of 0.4 millimeters, can suck up around 10 microliters of blood from a small drop of blood, as flows when the fingertip or earlobe is pricked. The interior wall surface of this very simple, unpacked extraction pipette is around 100 square millimeters. With a monomolecular covering, it can hold around 100 nanograms of a substance with a molecular weight of 1,000 Daltons; this corresponds to around 100 picomoles. If the extracted substances are heavier, more can be accommodated because the layer of molecules is thicker. This maximum extraction quantity has, however, to be shared by all 50 to 200 substances, which are generally extracted in a single extraction process. Since the detection limit in the mass spectrometer is around ten femtomoles, this simple, unpacked extraction pipette can quite easily cover the concentration range of interest.

Figure 1 illustrates an extraction pipette equipped with packing material; the one shown here even has two types of packing material in two capillary zones (3) and (4). In this example, there is an empty space (1) with a capacity of 10 microliters in front of the first packed capillary zone (3), This empty space (1) is initially filled with whole blood. The blood is then drawn through the two packed capillary zones (3) and (4) into a further empty space (6) by releasing the pipette bulb (7), and again forced back into the empty space (1). This process can be repeated a fixed number of times. The packing in the packed capillary zones (3) and (4) can consist of plastic foam, felt, fritted material, or packed particles as in chromatographic columns, for example, so that the actively binding surface can easily be increased ten to a thousand fold compared with an unpacked extraction pipette. The packings of the two packed capillary zones (3) and (4) have different types of actively binding layers each, in order to be able to carry out two different types of extraction from only one drop of blood. For example, it is possible to equip one packed capillary zone with a hydrophobic coating made of covalently bound alkanes, each being eight carbon atoms in length ("C8"), for extracting hydrophobic proteins and other hydrophobic substances, and the other packed capillary zone with a weakly binding anion exchanger ("WAX" = weak anion exchanger), for extracting anion proteins and other charged substances. The plastic capillary of the pipette can later be cut up in the mass spectrometric laboratory in order to elute and measure the individual protein extractions separately. The removal of the substances of interest from the surface layers can usually be performed by elution with strongly polar organic solvents.

The extraction pipette is equipped with unique identifiers, the different packed capillary zones (3), (4) each having such identifiers to permit identification even after the pipette has been cut up. The identifiers can consist of printed barcodes on the outside of the pipette body, adhesive printed tags (2), (5) made of paper or plastic with barcodes or other information, or chips holding information which can be electronically read out (RFID = radio frequency identification).

Pipettes of this type may be equipped also with more then two extraction zones to measure more then two sets of substances of interest.

The blood can be brought into good and intimate contact with the interior surfaces by repeatedly drawing it up a fixed number of times. The blood can then be removed and a washing liquid, preferably also ready-made, can be drawn up. Repeated washing leads to the complete removal of the blood and all non-immobilized substances. The extraction pipette can now be sealed with the cap and sent to the mass spectrometric laboratory. Depending on the method specification, the washing liquid filled last can remain in the extraction pipette; or the pipette can be filled with a special transportation liquid, which also may come ready-made; or the drained extraction pipette, moist or dried, then preferably filled with inert gas, can be shipped. The washing liquid or transportation liquid can contain antibiotic substances, for example lead azide, to avoid any biological activity from microorganisms coming with the blood.

Moreover, there can be one or more substances present in the extraction pipette which can immediately dissolve in the blood. Some of the substances may also be extracted by the surface layers and immobilized, and may serve as a concentration reference for the mass spectrometric measurement. Where possible, these reference substances should not be already immobilized on the surface layers before the blood sample is filled in but should initially be able to freely dissolve in the blood that has been drawn up so that they can later reflect the extracted quantity including the degree of immobilizing. Favorably, they could be present as an easily solvable thin film at the walls of the empty space (1). Other substances may avoid clotting of the blood, like heparin, or may help to immobilize the substances of interest, for instance by reducing the viscosity of the blood sample.

In the mass spectrometry laboratory, the reversibly immobilized substances can be removed from the extraction pipette or its pieces simply by taking them up with around one microliter of a strong organic solvent, for example acetone, acetonitrile or methanol, and transferred to the mass spectrometer for analysis and measurement of the substance profile. In mass spectrometers with electrospray ionization, the solution can be offered by nano-electrospray needles or by pre-separation with liquid chromatography (HPLC). For MALDI mass spectrometers, the eluent is placed onto the mass spectrometric sample support. The quantity of solvent is not critical since it will subsequently be vaporized. The solvent can be already provided with a matrix substance, as is required for ionization in the MALDI mass spectrometer. Alternatively, the matrix substance can be already on the sample support or be added afterwards. This process can easily be automated. It is also simple to read out a barcode identifier located on the capillary pipette so that sample mix-ups can be excluded as far as possible.

In the MALDI mass spectrometer, the dried sample on the sample support is then bombarded with flashes of laser light in the ion source of the mass spectrometer; the ions created are separated according to their mass by means of their time of flight in the time-of-flight mass spectrometer; they are then detected by an ion detector, and measured in terms of their relative quantities with respect to each other or to the reference substances. This process of ionization using matrix-assisted laser desorption (MALDI)) provides only singly charged, intact ions of the protein molecules; the mass spectrum is thus a true representation of the protein profile. On the other hand, the eluent can be added to a mass spectrometer with electrospray ion source (ESI) either directly or separated again by a chromatograph. This type of ionization also supplies multiply charged ions of the analyte molecules, however; the mass spectrum is therefore more difficult to analyze. It is for this reason that MALDI mass spectrometry is generally preferred.

A further, likewise very simple embodiment of the invention consists in taking a blood sample by inserting a needle into the vein, as it is a standard procedure in every medical office or doctor's surgery. This involves filling an evacuated blood sampling vessel with several tens of milliliters of blood. If the extraction of the substances of interest can be carried out from whole blood which is to be preferred, then this vessel is already prepared for extraction. This is achieved by an active layer on the interior vessel surface or again by using packing pellets or packing material with actively binding surfaces.

If the extraction is carried out by an active layer on the interior surface of the sampling vessel, the method is similar to the one described above with capillary pipettes. Care must be taken that the blood comes into very intimate contact with the walls of the vessel. This can be achieved by frequently turning it over, possibly in a small automatic turning device, or particularly by filling the sampling vessel with suitable packing pellets. The packing pellets sink each time the vessel is turned over and thus continually stir the blood. Magnetic stirring bodies can generate a continuous mixing motion in a magnetic rotating field.

The packing pellets themselves can also be equipped with active surfaces for extraction. In this case, packing pellets with different shapes or colors can even be equipped with different types of layers to extract different sets of substances of interest. These may include actively binding surfaces for the extraction of globulins and albumins which brings about a depletion of these highly concentrated and sometimes interfering proteins.

The packing pellets can be macroscopically large or microscopically small. Microparticles with a magnetic core can be collected on the wall of the vessel or drawn through the liquid particularly well using simple permanent magnets. The microparticles are characterized by a particularly large active surface, but they are not that good for whole blood extraction because of the risk of coagulation with the blood corpuscles.

An alternative to these extractions from whole blood is extraction from the blood serum or from blood plasma, which can also generally be obtained from whole blood in every doctor's practice by well-known standardized methods. The subsequently performed extractions are likewise carried out in ready-made vessels. Here, as well, the interior surfaces of the vessels, or packing pellets or other packing material placed in the vessels, can be used for the extraction. Magnetic microbeads are very good here, for example.

The above-described extraction pipettes can, of course, also be used for extraction from blood serum or blood plasma.

A particular diagnostic method consists in initially allowing the characteristic enzymes that are formed with certain diseases to act, after the blood has been taken, in the whole blood, the blood serum or the blood plasma without the addition of enzyme inhibitors. The method specification contains precise information as to the temperature and duration of storage, which is generally from some ten minutes to around 48 hours. Only then is the whole blood, the blood serum or the blood plasma filled into the vessels for the extraction, and it is particularly the digest peptides produced by the enzymes which are extracted. These can often be extracted particularly well with hydrophobic surfaces which are coated with covalently attached alkane molecules with a length of 18 carbon atoms ("C 18"). This means that enzymes which evade any normal mass spectrometric measurement of a substance profile because their concentration is much too low can be detected by means of their digest products. This very promising method is particularly assisted by the invention described here because the work of the enzymes has to occur directly after the blood has been taken, and possibly after removal of the blood corpuscles and clotting substances, in a well-controlled fashion while still in the doctor's office.

There are, however, still other methods which can also reliably measure substances at very low concentration. These require that the substances, e.g. the proteins of interest, are precisely known. They can then be specifically extracted ("fished out") from the blood serum or blood plasma by permanently covalently bound, i.e. not just reversibly immobilized, monoclonal or polyclonal antibodies for precisely these substances. The antibodies for extracting a series of interesting proteins can be located on the surface of small magnet beads, for example. Since only a few proteins are cleanly extracted here, the protein profile measured mass spectrometrically in this way is likewise very clean; it is thus well possible to measure concentrations in quantities down to 100 femtomoles (corresponding to approx. 10⁻¹⁰ to 10⁻⁹ grams). It is thus possible to measure proteins (or other fishable substances) with concentrations of only 10⁻⁹ to 10⁻⁸ per cent from only ten milliliters of blood (10 grams) if these proteins can be extracted completely. Work on these methods is in progress. It is not yet known if the theoretical predictions actually occur. Neither is it known to what extent biomarkers can actually be found in these concentration ranges.

Very recently it has also become possible to achieve the effect of the antibodies, which have molecular weights in the region between 150,000 and 190,000 Daltons, using computer-modeled peptides in the seize of only 20 amino acids (only around 2,400 Daltons), thus replacing the expensive antibodies by peptides which can be synthesized inexpensively. There is, however, a risk of increased cross-reactivity, but this is easily recognized with a mass spectrometer. Other particularly effective interaction partners are also known, such as lectins, metal chelates for phosphate bonding (IMAC), protein nucleic acids (PNA), oligonucleotides, inhibitors, receptors and ligands.

In order to measure the concentration ratios of different protein derivatives in a liquid sample, a quantity of a suspension of microparticles may be pipetted into the sample, the microparticles being coated with permanently bound capture molecules. The microparticles are preferably magnetizable. Suspensions of magnetic beads 900 nanometers in diameter have already proven to be very successful for other applications; suspensions of these beads stay usable for a long time. The capture molecules can be monoclonal or polyclonal antibodies or molecules of a similar specificity, for example. Special care must be taken here that the microparticles are not loaded to saturation for any of the protein derivatives to be measured since, otherwise, the concentration ratios can no longer be measured correctly.

The small particles are then separated from the liquid. Small magnetizable particles can be drawn to the wall of the vessel by a strong permanent magnet, for example. To this end, the vessel should not be too large since the effect of the magnet only extends some five to ten millimeters. Careful stirring or shaking also helps here to bring all the particles slowly within range of the magnet and thus to finally capture them in clusters on the wall.

The particle accumulations adhering to the wall or sedimented are then freed from the sample solution by draining or pipetting and a washing liquid is added. The particles are washed by removing the magnet and by stirring. The washing process can be repeated several times where necessary. Finally, an elution liquid is added to the accumulation of particles, which is now largely free of liquid, in order to separate the proteins from the antibodies or the other types of capture molecules. These elution liquids are generally strong, polar organic solvents such as acetone, acetonitrile or alcohols. The elution liquids with the proteins are then fed to the mass spectrometric measurement.

With knowledge of this invention, those skilled in the art can develop further embodiments of the method and equipment. All these embodiments should be included in the basic idea of the invention.

## Claims

1. Method of preparing samples for a mass spectrometric analysis of substances of interest extracted from a subject's blood, whereby the blood is taken from the subject at a first location, and the mass spectrometric analysis is performed at a second location, comprising the steps
a) providing an extraction vessel internally comprising a surface layer capable to reversibly immobilize substances of interest,
b) obtaining a fresh blood sample from the subject at the first location,
c) immediately filling the fresh blood sample into the extraction vessel, thereby performing the extraction by immobilizing the substances of interest on the surface layer,
e) removing the blood sample and washing the immobilized substances of interest with a washing fluid,
f) transporting the vessel with the immobilized substances of interest from the first location of to the second location, and
g) removing the substances of interest from the surface layer and analyzing the substances of interest by mass spectrometry.

2. Method according to Claim 1, wherein the extraction vessel is filled with whole blood which it is taken from the subject and the extraction is performed on the whole blood.

3. Method according to Claim 2, wherein the extraction vessel is a pipette into which a drop of whole blood is drawn.

4. Method according to Claim 3, wherein the drop of whole blood is generated by pricking the fingertip or earlobe.

5. Method according to Claim 1, wherein the extraction vessel is filled with blood serum or blood plasma, immediately obtained from the fresh blood after taking it, and the extraction is performed on one of these liquids.

6. Method according to Claim 1, wherein the blood sample is moved inside the vessel to bring the substances of interest in contact with the surface layers.

7. Method according to Claim 1, wherein the extraction is carried out by reversible immobilization of the substances of interest on hydrophobic layers, on layers with anion or cation exchangers, on metal layers of different types, or layers of antibodies, or layers of synthetic substance-specific bonding molecules.

8. Method according to Claim 1, wherein different types of surface layers on distinguishable packing pellets or different surface layers in distinguishable zones of the vessel perform different types of extractions for different sets of substances of interest simultaneously.

9. Method according to Claim 1, wherein one or more internal reference substances for one or more sets of substances are added to the blood sample for quantifying purposes.

10. Method according to Claim 1, wherein one or more liquid or solid substances are added to the blood to avoid clotting or to help immobilization.

11. Method according to one of Claims 9 or 10, wherein the added substances are already contained in the extraction vessel before the blood sample is filled in.

12. Tubes for taking blood samples in doctor's practices, evacuated in a standard manner and equipped with a piercable membrane,
*wherein* the tubes internally comprise at least one type of surface layer for the reversible immobilization of at least one set of substances.

13. Extraction pipette for the extraction of one or more sets substances of interest from whole blood,
*comprising*
a) a capillary as the pipette body,
b) an attached rubber bulb to draw up and squeeze out blood and washing liquids, and
c) at least one type of surface layer in the interior of the pipette capillary for the reversible immobilization of at least one set of substances of interest.

14. Extraction pipette according to Claim 10, wherein at least two different types of surface layers in at least two distinguishable capillary zones are available for the extraction of at least two different sets of substances of interest.

15. Extraction pipette according to Claim 10, wherein it is equipped with identifiers for assigning the sets of substances of interest to the type of extraction and origin of blood sample.
